# EUROPEAN PATENT APPLICATION

(11) **EP 4 792 664 A1**
(43) Date of publication of application: **19.08.2026**
(21) Application number: 24877326.9
(22) Date of filing: 15.10.2024
(51) Int. Cl.: A23L 33/10, A23L 27/50, A23L 33/125, C12N 1/20, C12P 7/52, C12P 7/56

(54) **GROWTH PROMOTER FOR BUTYRIC ACID-PRODUCING BACTERIA, AND BUTYRIC ACID OR LACTIC ACID PRODUCTION AMOUNT INCREASING AGENT**

(30) Priority: 13.10.2023 JP 2023177858; 13.10.2023 JP 2023177857
(71) Applicant: Kikkoman Corporation, Noda-shi, Chiba 278-8601 (JP); National University Corporation Chiba University, Chiba-shi, Chiba 263-8522 (JP)
(72) Inventor: HAYASHI, Kanako, Noda-shi, Chiba 278-8601 (JP); GOTO, Yoshiyuki, Chiba-shi, Chiba 260-8670 (JP)
(74) Representative: Mewburn Ellis LLP
(86) International application number: PCT/JP2024/036716
(87) International publication number: WO 2025/079732

(57) **Abstract**

The present invention relates to a growth promoter for butyrate-producing bacteria, which contains dark soy sauce or a soy sauce-like seasoning liquid, or a precipitate thereof with ethanol. The present invention also relates to a butyric acid or lactic acid production enhancer, which contains dark soy sauce or a soy sauce-like seasoning liquid, or a precipitate thereof with ethanol.

## Description

### Technical Field

The present invention relates to a growth promoter for butyrate-producing bacteria. The present invention also relates to a butyric acid or lactic acid production enhancer.

### Background Art

Short-chain fatty acids such as butyric acid, acetic acid and propionic acid are known to have various positive effects on the bowel. For example, Non Patent Literature 1 indicates that short-chain fatty acids maintain the barrier function of the bowel, reduce pH of the inside of the bowel to suppress proliferation of pathogenic bacteria, and have an anti-inflammatory effect, an immunomodulatory function, and an effect of suppressing obesity by increasing insulin sensitivity and improving maintenance of a feeling of fullness, for example.

Among the positive effects of short-chain fatty acid on the bowel, the maintenance of the barrier function of the bowel is very important. The bowel has a function of absorbing substances necessary for maintaining living organisms, such as water and nutrient components, as well as a function of appropriately eliminating unnecessary substances such as detrimental substances and pathogenic cells, and hence an important role as a barrier that restricts entry of foreign matter. If the barrier function of the bowel is lost and the bowel tract is damaged, foreign matter enters into mucosal membranes, triggers an excessive immune reaction, and causes inflammation, and further, the foreign matter joins the bloodstream, which leads to a state of systemic inflammation.

Non Patent Literature 2 indicates that a bowel in a state where the bowel is perforated, so that leakage into the blood occurs is called a leaky gut, and the leaky gut is considered a problem because it causes chronic inflammation. Therefore, the barrier function of the bowel is essential for animals to maintain their health.

Here, Non Patent Literature 1 and Non Patent Literature 2 indicate that among short-chain fatty acids, butyric acid particularly enhances the barrier function of the bowel by increasing tight junction, and producing mucin and antimicrobial peptides. Therefore, butyric acid is expected to restrict passage of microorganisms and foreign matter into the lamina propria, and prevent the leaky gut.

Further, Non Patent Literature 3 indicates that butyric acid is essential for differentiation of regulatory T cells (Treg) that are effective for suppression of allergy and inflammatory bowel diseases (IBDs), and Non Patent Literature 4 indicates that consumption of oxygen in the bowel increases when butyric acid is metabolized by bowel tract epithelial cells. Therefore, butyric acid is expected to not only suppress IBD and enhance the barrier function of the bowel, but also have an effect of maintaining the immune response in the bowel, and an effect of improving bowel microbiota by maintaining an anaerobic condition in the bowel to increasing the number of beneficial bacteria such as bifidobacteria.

Thus, in the bowel, butyric acid plays various important roles such as enhancement of the barrier function of the bowel.

Here, dietary fibers are consumed by bacteria in the bowel, or fructose, oligofructose or inulin is consumed by butyrate-producing bacteria such as Eubacterium rectale (currently called Agathobacter rectalis) to produce butyric acid (see Non Patent Literature 5).

Among butyrate-producing bacteria, Agathobacter rectalis settles on mucous layers covering the bowel tract epithelium, and thus produces butyric acid near bowel tract epithelial cells, so that a physiological action such as enhancement of the barrier function of the bowel by butyric acid can be efficiently exhibited (see Non Patent Literature 6). Since in IBD patients, a decreased amount of microorganism groups including Agathobacter rectalis is confirmed, involvement of Agathobacter rectalis in suppression of IBD has been suggested (see Non Patent Literature 7).

Further, Patent Literature 1 discloses a growth promoter for butyrate-producing bacteria and an agent for enhancing the amount of butyric acid in the bowel, which are intended to effectively enhancing the amounts of butyrate-producing bacteria and butyric acid in the bowel, and contain, as an active ingredient, 1-kestose that is a type of oligosaccharide.

### Citation List

### Patent Literature

Patent Literature 1: International Publication No. 2017/159643

### Non-Patent Literature

Non-Patent Literature 1: Riviere et al. Bifidobacteria and Butyrate-Producing Colon Bacteria: Importance and Strategies for Their Stimulation in the Human Gut. Frontiers in Microbiology. 2016, 7, Article 979.
Non-Patent Literature 2: KOBAYASHI Kyosuke/ASAMI Yukio, Effect of Yogurt on Intestinal Barrier Function. Jpn. J. Food Microbiol. 2019, 36 (1), 32-35.
Non-Patent Literature 3: Furusawa et al. Commensal microbe-derived butyrate induces the differentiation of colonic regulatory T cells. Nature. 2013, 504, 446-450.
Non-Patent Literature 4: SUZUKI Takuya. Role of intestinal tight junction barrier and the regulation by dietary factors. Milk Science. 2020, 69 (3), 180-186.
Non-Patent Literature 5: F. Moens & De Vuyst. Inulin-type fructan degradation capacity of Clostridium cluster IV and XIVa butyrate-producing colon bacteria and their associated metabolic outcomes. Beneficial Microbes. 2017, 8(3), 473-490.
Non-Patent Literature 6: P Van den Abbeele et al. Butyrate-producing Clostridium cluster XIVa species specifically colonize mucins in an in vitro gut model. The ISME Journal. 2013, 7, 949-961.
Non-Patent Literature 7: Heinken et al. Systematic assessment of secondary bile acid metabolism in gut microbes reveals distinct metabolic capabilities in inflammatory bowel disease. Microbiome. 2019, 7, Article 75.

### Summary of Invention

### Technical Problem

A problem to be solved by the present invention is to provide a novel component capable of promoting growth of butyrate-producing bacteria. Another problem to be solved by the present invention is to provide a novel component capable of enhancing the production of butyric acid or lactic acid.

### Solution to Problem

The present inventors have conducted intensive studies for solving the above problems, and resultantly discovered that dark soy sauce, a precipitate of dark soy sauce with ethanol, a soy sauce-like seasoning liquid, or a precipitate of a soy sauce-like seasoning liquid with ethanol promotes growth of butyrate-producing bacteria, and enhances the production of butyric acid or lactic acid, leading to completion of the present invention.

That is, the present invention is as follows.
(1)
   A growth promoter for butyrate-producing bacteria, comprising dark soy sauce or a soy sauce-like seasoning liquid, or a precipitate thereof with ethanol.
(2)
   The growth promoter for butyrate-producing bacteria according to (1), comprising a polysaccharide derived from dark soy sauce or a soy sauce-like seasoning liquid.
(3)
   A food composition comprising the growth promoter for butyrate-producing bacteria according to (1) or (2).
(4)
   A food composition for promoting growth of butyrate-producing bacteria, comprising dark soy sauce, a soy sauce-like seasoning liquid, or a precipitate thereof with ethanol.
(5)
   A method for promoting growth of butyrate-producing bacteria using dark soy sauce or a soy sauce-like seasoning liquid, or a precipitate thereof with ethanol.
(6)
   A butyric acid or lactic acid production enhancer comprising dark soy sauce or a soy sauce-like seasoning liquid, or a precipitate thereof with ethanol.
(7)
   The butyric acid or lactic acid production enhancer according to (6), comprising a polysaccharide derived from dark soy sauce or a soy sauce-like seasoning liquid.
(8)
   A food composition comprising the butyric acid or lactic acid production enhancer according to (6) or (7).
(9)
   A food composition for enhancing the production of butyric acid or lactic acid, comprising dark soy sauce or a soy sauce-like seasoning liquid, or a precipitate thereof with ethanol.
(10)
   A method for enhancing the production of butyric acid or lactic acid using dark soy sauce or a soy sauce-like seasoning liquid, or a precipitate thereof with ethanol.

In (1) to (10) above
may be a growth promoter for butyrate-producing bacteria, comprising dark soy sauce or a precipitate of dark soy sauce with ethanol,
may be a method for promoting growth of butyrate-producing bacteria using dark soy sauce or a precipitate of dark soy sauce with ethanol,
may be a butyric acid or lactic acid production enhancer comprising a soy sauce-like seasoning liquid or a precipitate of a soy sauce-like seasoning liquid with ethanol, and
may be a method for enhancing the production of butyric acid or lactic acid using a soy sauce-like seasoning liquid or a precipitate of a soy sauce-like seasoning liquid with ethanol.

In addition, in the respective aspects above, the butyrate-producing bacteria may be Agathobacter rectalis.

The present invention may be as follows.
(11)
   Dark soy sauce or a soy sauce-like seasoning liquid, or a precipitate thereof with ethanol for use in promotion of growth of butyrate-producing bacteria.
(12)
   A food composition comprising dark soy sauce or a soy sauce-like seasoning liquid, or a precipitate thereof with ethanol, for use in promotion of growth of butyrate-producing bacteria.
(13)
   Use of dark soy sauce or a soy sauce-like seasoning liquid, or a precipitate thereof with ethanol to promote growth of butyrate-producing bacteria.
(14)
   Use of a food composition comprising dark soy sauce or a soy sauce-like seasoning liquid, or a precipitate thereof with ethanol, to promote growth of butyrate-producing bacteria.
(15)
   Use of dark soy sauce or a soy sauce-like seasoning liquid, or a precipitate thereof with ethanol in manufacturing of a growth promoter for butyrate-producing bacteria.
(16)
   Use of dark soy sauce or a soy sauce-like seasoning liquid, or a precipitate thereof with ethanol in manufacturing of a food composition for promoting growth of butyrate-producing bacteria.

In (11) to (16) above,
dark soy sauce or a precipitate of dark soy sauce with ethanol is preferably used,
a polysaccharide derived from dark soy sauce may be contained, and/or
the butyrate-producing bacteria may be Agathobacter rectalis.

The present invention may be as follows.
(21)
   Dark soy sauce or a soy sauce-like seasoning liquid, or a precipitate thereof with ethanol for use in enhancing the production of butyric acid or lactic acid.
(22)
   A food composition comprising dark soy sauce or a soy sauce-like seasoning liquid, or a precipitate thereof with ethanol, for use in enhancing the production of butyric acid or lactic acid.
(23)
   Use of dark soy sauce or a soy sauce-like seasoning liquid, or a precipitate thereof with ethanol to enhance the production of butyric acid or lactic acid.
(24)
   Use of a food composition comprising dark soy sauce or a soy sauce-like seasoning liquid, or a precipitate thereof with ethanol, to enhance the production of butyric acid or lactic acid.
(25)
   Use of dark soy sauce or a soy sauce-like seasoning liquid, or a precipitate thereof with ethanol in manufacturing of a butyric acid or lactic acid production enhancer.
(26)
   Use of dark soy sauce or a soy sauce-like seasoning liquid, or a precipitate thereof with ethanol in manufacturing of a food composition for enhancing the production of butyric acid or lactic acid.

In (21) to (26) above,
A soy sauce-like seasoning liquid or a precipitate of a soy sauce-like seasoning liquid with ethanol is preferably used,
a polysaccharide derived from a soy sauce-like seasoning liquid may be contained, and/or
the butyrate-producing bacteria may be Agathobacter rectalis.

The invention may include a therapeutic invention, but may include a non-therapeutic invention. That is, an invention of a therapeutic or non-therapeutic method may be included, an invention of therapeutic or non-therapeutic use may be included, and an invention of a therapeutic or non-therapeutic thing may be included.

### Advantageous Effect of Invention

According to the present invention, a novel component capable of promoting growth of butyrate-producing bacteria can be provided. According to the present invention, a novel component capable of enhancing the production of butyric acid or lactic acid can also be provided.

### Brief Description of Drawings

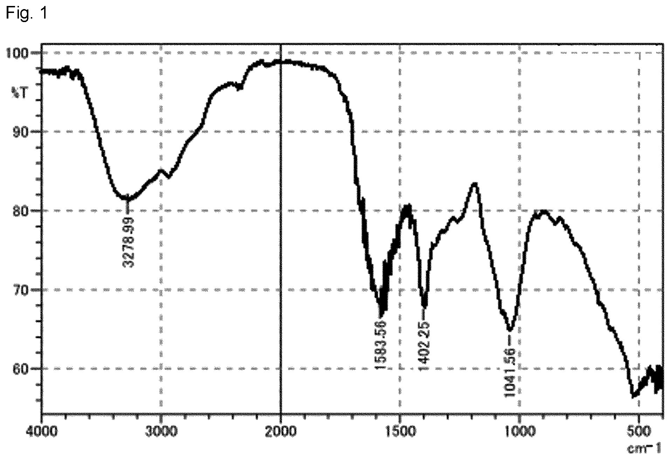

[Figure 1] Figure 1 shows the results of measuring an infrared absorption spectrum of a precipitate of a soy sauce-like seasoning liquid with ethanol by Fourier transform infrared spectroscopy (FTIR) using an infrared spectrophotometer.

### Description of Embodiments

Hereinafter, modes for carrying out the present invention (hereinafter, referred to as "the present embodiment") will be described in detail. Note that the present invention is not limited to the present embodiment below.

The present embodiment provides a growth promoter for butyrate-producing bacteria which contains dark soy sauce or a soy sauce-like seasoning liquid, or a precipitate thereof with ethanol, that is, one or more selected from the group consisting of dark soy sauce, a precipitate of dark soy sauce with ethanol, a soy sauce-like seasoning liquid, and a precipitate of a soy sauce-like seasoning liquid with ethanol.

In the present embodiment, the term "dark soy sauce" refers to, for example, soy sauce corresponding to "dark soy sauce" defined in Japanese Agriculture Standards. The total nitrogen content may be 0.95 g/100 mL or more. In the present embodiment, the "dark soy sauce" is made by, for example, adding salt water to koji obtained by addition of aspergillus to raw materials such as soy and wheat, followed by fermentation and aging.

In the present embodiment, the term "soy sauce-like seasoning liquid" refers to a seasoning liquid that is used for the same application as "soy sauce" defined in Japanese Agriculture Standards.

The soy sauce-like seasoning liquid is not particularly limited, but examples thereof include soy sauce-like seasoning liquids described in Japanese Patent Laid-Open No. 2016-086700 and Japanese Patent Laid-Open No. 2016-059382.

Although there is no particular limitation, the soy sauce-like seasoning liquid can be manufactured in the following manner, for example.

Aspergillus is added to a grain raw material, and cultured at 25 to 40°C for 2 to 3 days to obtain soy sauce koji. To the soy sauce koji, salt water is added at a salt concentration of less than 4% (w/v), and the mixture is warmed for decomposition at 25 to 57 °C for 0 to 48 hours to obtain unrefined soy sauce. Sterilization treatment is performed on the unrefined soy sauce, and 1 × 10⁵ or more cells of yeast are then added per g of the unrefined soy sauce, followed by fermentation at 15 to 45°C for 1 to 90 days. The unrefined soy sauce after fermentation is subjected to conventional production processes such as pressing, heating, clarification and filtration to obtain a soy sauce-like seasoning liquid.

The soy sauce-like seasoning agent may be obtained by adding salt before pressing of the unrefined soy sauce after fermentation, before heating, before clarification, before treatment such as filtration, or after completion of all processes.

The grain raw material, aspergillus, yeast and the like used for obtaining the soy sauce-like seasoning liquid are not particularly limited. Those that have been heretofore known may be used.

The grain raw material is not particularly limited, but examples thereof include protein materials such as whole soy, defatted soy, soy protein, wheat gluten, peas, broad beans and red beans, and starch raw materials such as wheat, barley, rye, bran, rice, rice bran, corn and starch bran.

The aspergillus is not particularly limited, but examples thereof include Aspergillus sojae, and Aspergillus oryzae.

The yeast is not particularly limited, but examples thereof include Saccharomyces cerevisiae, Zygosaccharomyces rouxii, and Kluyveromyces marxianus.

The unrefined soy sauce prepared may be adjusted to a pH of 3.0 to 7.0 using organic acids such as lactic acid, acetic acid, malic acid, citric acid, gluconic acid and adipic acid, and subjected to a next process.

An example of a specific method for manufacturing a soy sauce-like seasoning liquid is shown below. To a solid medium containing equal amounts of soy modified by heating and wheat, spores of Aspergillus sojae are added as aspergillus, and koji production is conventionally performed at 25 to 40°C for 42 hours to obtain soy sauce koji. Next, the soy modified by heating and the soy sauce koji are mixed at a 1 : 2 or more ratio, salt water is mixed at a salt concentration of less than 4% (w/v), and the mixture is stirred for 24 hours at 50°C and 100 rpm for digestion. Cooling to room temperature is performed, and lactic acid is added to obtain unrefined soy sauce adjusted to a pH of 4.0 to 5.0. Thereafter, sterilization treatment is performed at 121°C for 5 minutes, and at the same time, the enzyme in the unrefined soy sauce is neutralized. Soy sauce yeast **Zygosaccharomyces** rouxii is added at 1 × 10⁷ cells/g of unrefined soy sauce, and fermentation is performed for 5 days with air allowed to pass on a timely basis at an unrefined soy sauce product temperature of 25°C in accordance with a conventional method. Salt is mixed with the resulting unrefined soy sauce at a salt concentration of 10%, the mixture is then press-filtered, and the resulting filtered product is heated at 80°C for 30 minutes, put into a clarification tank, and clarified (suspended solids are removed) over 3 days to obtain a soy sauce-like seasoning liquid which is clear and has good flavor.

In the present embodiment, the "precipitate of dark soy sauce with ethanol" and the "precipitate of a soy sauce-like seasoning liquid with ethanol" are precipitates obtained by adding ethanol to the "dark soy sauce" and the "soy sauce-like seasoning liquid", respectively.

Although there is no particular limitation, the precipitate of dark soy sauce with ethanol or the precipitate of a soy sauce-like seasoning liquid with ethanol can be obtained by a method known to those skilled in the art. For example, by adding ethanol to dark soy sauce or a soy sauce-like seasoning liquid at a final ethanol concentration of 10 to 99% (v/v), and allowing the mixture to stand at a temperature of -80°C to 30°C for 1 second to 30 minutes, a precipitate can be obtained. After addition of ethanol, the mixture may be appropriately centrifuged to obtain a precipitate. The resulting precipitate may be dialyzed with a cellulose-based dialysis membrane or the like to select a precipitate having a desired molecular size. The precipitate (including a precipitate after dialysis) dissolved in a solvent such as water may be used in the present embodiment.

The ethanol for use in ethanol precipitation is not particularly limited, but ethanol with a concentration of 90% or more may be used.

The growth promoter for butyrate-producing bacteria in the present embodiment may contain only one selected from the group consisting of dark soy sauce, a precipitate of dark soy sauce with ethanol, a soy sauce-like seasoning liquid, and a precipitate of a soy sauce-like seasoning liquid with ethanol, or may contain two, three or four selected from the group consisting of dark soy sauce, a precipitate of dark soy sauce with ethanol, a soy sauce-like seasoning liquid, and a precipitate of a soy sauce-like seasoning liquid with ethanol. The growth promoter for butyrate-producing bacteria may contain only any of dark soy sauce, a precipitate of dark soy sauce with ethanol, a soy sauce-like seasoning liquid, and a soy sauce-like seasoning liquid.

In the present embodiment, the dark soy sauce, the precipitate of dark soy sauce with ethanol, the soy sauce-like seasoning liquid or the precipitate of a soy sauce-like seasoning liquid with ethanol may contain a polysaccharide. In this case, the present embodiment also provides a growth promoter for butyrate-producing bacteria, which contains a polysaccharide derived from dark soy sauce or a soy sauce-like seasoning liquid.

The polysaccharide derived from dark soy sauce or a soy sauce-like seasoning liquid can be analyzed by a method known to those skilled in the art, and can be analyzed by, for example, Fourier transform infrared spectroscopy (FTIR) or an HPLC method.

The polysaccharide is a saccharide formed by large number of monosaccharide molecules bound together via a glycoside bond. The polysaccharide in the present embodiment may have a molecular weight of more than 500 Da. The polysaccharide derived from dark soy sauce or a soy sauce-like seasoning liquid is not particularly limited, but may be, for example, a polysaccharide that is understood by referring to Japanese Patent Laid-Open No. 2012-036100 and Japanese Patent Laid-Open No. 2005-179315. For example, since the polysaccharide that remains to the end in the soy sauce without being decomposed in the process of manufacturing is polygalacturonic acid (KIKUCHI Tadaaki, Changes of Soybean Cell Wall Polysaccharides during Soy Sauce Fermentation, Journal of Japan Society for Bioscience, Biotechnology and Agrochemistry, 1976, 50(6), 273-277.), the polysaccharide derived from dark soy sauce or a soy sauce-like seasoning liquid may contain polygalacturonic acid as a main component. Further, from the constituent saccharide composition of the polysaccharide derived from soy (NAKAMURA Akihiro, Development of Soybean Soluble Polysaccharide Derived from "Okara", and Application as a Functional Food Ingredient, Journal of Japanese Society for Food Science and Technology, 2011, 58(11), 559-566, or Japanese Patent Laid Open No. 2012-036100), and the constituent saccharide composition of the polysaccharide derived from soy sauce (KIKUCHI Tadaaki, Changes of Soybean Cell Wall Polysaccharides during Soy Sauce Fermentation, Journal of Japan Society for Bioscience, Biotechnology and Agrochemistry, 1976, 50(6), 273-277.), constituent saccharides of the polysaccharide in the present embodiment may contain, in addition to galacturonic acid, galactose, arabinose, rhamnose, fucose, xylose, glucose, mannose and ribose. A part of arabinogalactan composed of arabinose and galactose that are constituent saccharides may be bound to polygalacturonic acid that is a polysaccharide included in the present embodiment.

The polysaccharide derived from dark soy sauce or a soy sauce-like seasoning liquid is not particularly limited, but may be, for example, a polysaccharide that is understood by referring to Japanese Patent Laid Open No. 2012-036100 and Japanese Patent Laid Open No. 2005-179315.

As a mode of the present embodiment, for enhancing the amount of butyric acid in the bowel, it is important that polysaccharides such as dietary fibers are metabolized by butyrate-producing bacteria and the like to enhance the number of butyrate-producing bacteria. As a result of the enhanced butyrate-producing bacteria, the production of butyric acid can be expected to enhance. That is, it can be expected that metabolism of polysaccharides such as dietary fibers by butyrate-producing bacteria and the like promotes growth of butyrate-producing bacteria and the like, and the enhanced number of butyrate-producing bacteria and the like leads to an enhance in the production of butyric acid and the like. In the present embodiment, dark soy sauce, a precipitate of dark soy sauce with ethanol, a soy sauce-like seasoning liquid or a precipitate of a soy sauce-like seasoning liquid with ethanol may contain a polysaccharide, and thus the polysaccharide is metabolized by butyrate-producing bacteria and the like to produce butyric acid and the like. As a result of metabolizing the polysaccharide to enhance the number of butyrate-producing bacteria and the like that produce butyric acid and the like, the amount of butyric acid in the bowel can be expected to enhance, and the physiological functions of butyric acid and the like are expected to be exhibited in the bowel.

The growth promoter for butyrate-producing bacteria in the present embodiment, which contains one or more selected from the group consisting of dark soy sauce, a precipitate of dark soy sauce with ethanol, a soy sauce-like seasoning liquid, and a precipitate of a soy sauce-like seasoning liquid with ethanol, thus can promote growth of butyrate-producing bacteria.

Promoted growth of butyrate-producing bacteria may be evaluated based on proliferation of butyrate-producing bacteria. That is, when the number of butyrate-producing bacteria is larger in the case where one or more selected from the group consisting of dark soy sauce, a precipitate of dark soy sauce with ethanol, a soy sauce-like seasoning liquid, and a precipitate of a soy sauce-like seasoning liquid with ethanol are given to the butyrate-producing bacteria than in the case where one or more selected from the group consisting of dark soy sauce, a precipitate of dark soy sauce with ethanol, a soy sauce-like seasoning liquid, and a precipitate of a soy sauce-like seasoning liquid with ethanol are not given to the butyrate-producing bacteria, it can be evaluated that growth of butyrate-producing bacteria is promoted. When the number of butyrate-producing bacteria is larger and the production of butyric acid by the butyrate-producing bacteria is accordingly larger in the case where one or more selected from the group consisting of dark soy sauce, a precipitate of dark soy sauce with ethanol, a soy sauce-like seasoning liquid, and a precipitate of a soy sauce-like seasoning liquid with ethanol are given to the butyrate-producing bacteria than in the case where one or more selected from the group consisting of dark soy sauce, a precipitate of dark soy sauce with ethanol, a soy sauce-like seasoning liquid, and a precipitate of a soy sauce-like seasoning liquid with ethanol are not given to the butyrate-producing bacteria, it can also be evaluated that growth of butyrate-producing bacteria is promoted.

The proliferation of butyrate-producing bacteria can be evaluated by a method known to those skilled in the art, and can be evaluated by, for example, measuring the value of OD₆₀₀.

The butyrate-producing bacteria are not particularly limited, but examples thereof include Agathobacter rectalis, Faecalibacterium prausnitzii, Clostridium methylpentosum, Clostridium leptum, Clostridium sporosphaeroides, Clostridium cellulosi, Clostridium viride, Roseburia intestinalis, Roseburia inulinivorans, Roseburia faecis, Roseburia hominis, Eubacterium hallii, Anaerostipes caccae, Coprococcus comes, Coprococcus eutactus, Anaerostipes hadrus, Clostridium coccoides, Clostridium celerecrescens, Clostridium boltei, Clostridium oroticum, Clostridium nexile, Clostridium scindens, Clostridium butyricum, Blautia wexlerae, with Agathobacter rectalis being preferred.

Agathobacter rectalis that is preferred as butyrate-producing bacteria will be described as an example. Agathobacter rectalis has 52 glycosidases in a genome, which include, for example, βfructofuranosidase, α-arabinofuranosidase, β-xylosidase, exooligoxylanase, α-amylase, α- and β-glucosidase, α- and β-galactosidase, and cellulase. Other butyrate-producing bacteria also contain similar enzyme groups. Agathobacter rectalis contains a larger number of types of polysaccharide degradation enzymes over other butyrate-producing bacteria, thus metabolize polysaccharides, is expected to have high butyric acid production ability, and can be more preferred in the present invention. In the present embodiment, since dark soy sauce, a precipitate of dark soy sauce with ethanol, a soy sauce-like seasoning liquid or a precipitate of a soy sauce-like seasoning liquid with ethanol may contain a polysaccharide, the dark soy sauce, precipitate of dark soy sauce with ethanol, soy sauce-like seasoning liquid or precipitate of a soy sauce-like seasoning liquid with ethanol may be capable of promoting growth of butyrate-producing bacteria to enhance the production of butyric acid or lactic acid.

In the present embodiment, the butyric acid is a linear saturated carboxylic acid having 4 carbon atoms, and may be n-butyric acid, or isobutyric acid. In the present embodiment, the lactic acid is an α-hydroxylic acid having asymmetric carbon, and may be D-lactic acid or L-lactic acid, or may include both.

The aspect of the growth promoter for butyrate-producing bacteria according to the present embodiment is also applied to other embodiments which will be described below.

As another embodiment, a method for promoting growth of butyrate-producing bacteria using one or more selected from the group consisting of dark soy sauce, a precipitate of dark soy sauce with ethanol, a soy sauce-like seasoning liquid, and a precipitate of a soy sauce-like seasoning liquid with ethanol is also provided.

When the method for promoting growth of butyrate-producing bacteria according to the other embodiment is carried out for enhancing the number of butyrate-producing bacteria in the body (preferably in the bowel), or carried out in production of butyric acid by butyrate-producing bacteria, for example, proliferation of butyrate-producing bacteria can be promoted, and the production of butyric acid by butyrate-producing bacteria can also be enhanced by promoting proliferation of butyrate-producing bacteria. In the case where the method for promoting growth of butyrate-producing bacteria is carried out for enhancing the number of butyrate-producing bacteria in the body, the method may include a process of administering a growth promoter for butyrate-producing bacteria in the present embodiment to a target. The administration target is preferably a mammal, more preferably a human, a monkey, a cat, a pig, a horse, a cattle, a mouse, a rat, a guinea pig, a dog or a rabbit, and further more preferably a human. The dosage amount and the administration frequency of the growth promoter for butyrate-producing bacteria may be appropriately set according to a route of administration, and various conditions such as an age, a body weight and a symptom of the administration target.

As another embodiment, a food composition containing a growth promoter for butyrate-producing bacteria is also provided. That is, a food composition containing one or more selected from the group consisting of dark soy sauce, a precipitate of dark soy sauce with ethanol, a soy sauce-like seasoning liquid, and a precipitate of a soy sauce-like seasoning liquid with ethanol which promote growth of butyrate-producing bacteria is provided.

When a food composition contains a growth promoter for butyrate-producing bacteria, butyrate-producing bacteria exist in the food composition, or the food composition exists in an environment where butyrate-producing bacteria exist, and thus, a function of promoting growth of butyrate-producing bacteria by one or more selected from the group consisting of dark soy sauce, a precipitate of dark soy sauce with ethanol, a soy sauce-like seasoning liquid, and a precipitate of a soy sauce-like seasoning liquid with ethanol is exhibited. The function may be exhibited directly by the growth promoter for butyrate-producing bacteria, or the function may be exhibited through a food composition into which the growth promoter for butyrate-producing bacteria is incorporated.

In the present embodiment, the food composition may be a food composition for promoting growth of butyrate-producing bacteria, which contains butyric acid or lactic acid. The food composition containing a growth promoter for butyrate-producing bacteria may be a food composition for promoting growth of butyrate-producing bacteria, which contains a growth promoter for butyrate-producing bacteria.

As another embodiment, a butyric acid or lactic acid production enhancer containing one or more selected from the group consisting of dark soy sauce, a precipitate of dark soy sauce with ethanol, a soy sauce-like seasoning liquid, and a precipitate of a soy sauce-like seasoning liquid with ethanol is provided.

The butyric acid or lactic acid production enhancer, which contains one or more selected from the group consisting of dark soy sauce, a precipitate of dark soy sauce with ethanol, a soy sauce-like seasoning liquid, and a precipitate of a soy sauce-like seasoning liquid with ethanol, thus can enhance the production of butyric acid or lactic acid.

An enhanced production of butyric acid or lactic acid may be evaluated based on the production of butyric acid or lactic acid by butyrate-producing bacteria. That is, when the production of butyric acid or lactic acid by butyrate-producing bacteria is larger in the case where one or more selected from the group consisting of dark soy sauce, a precipitate of dark soy sauce with ethanol, a soy sauce-like seasoning liquid, and a precipitate of a soy sauce-like seasoning liquid with ethanol are given to the butyrate-producing bacteria than in the case where one or more selected from the group consisting of dark soy sauce, a precipitate of dark soy sauce with ethanol, a soy sauce-like seasoning liquid, and a precipitate of a soy sauce-like seasoning liquid with ethanol are not given to the butyrate-producing bacteria, it can be evaluated that the production enhances. For example, when the amount of butyric acid or lactic acid that one cell of butyrate-producing bacteria produces is larger in the case where one or more selected from the group consisting of dark soy sauce, a precipitate of dark soy sauce with ethanol, a soy sauce-like seasoning liquid, and a precipitate of a soy sauce-like seasoning liquid with ethanol are given to the butyrate-producing bacteria, it can be evaluated that the production enhances. For example, when the number of butyrate-producing bacteria is larger, and the production of butyric acid or lactic acid by butyrate-producing bacteria is accordingly larger in the case where one or more selected from the group consisting of dark soy sauce, a precipitate of dark soy sauce with ethanol, a soy sauce-like seasoning liquid, and a precipitate of a soy sauce-like seasoning liquid with ethanol are given to the butyrate-producing bacteria, it can be evaluated that the production enhances. Further, when the amount of butyric acid or lactic acid that one cell of butyrate-producing bacteria produces is larger, and the number of butyrate-producing bacteria is larger in the case where one or more selected from the group consisting of dark soy sauce, a precipitate of dark soy sauce with ethanol, a soy sauce-like seasoning liquid, and a precipitate of a soy sauce-like seasoning liquid with ethanol are given to the butyrate-producing bacteria, it can be evaluated that the production enhances.

The production of butyric acid or lactic acid can be measured by a method known to those skilled in the art, and can be measured by, for example, a HPLC method.

The butyric acid or lactic acid production enhancer may enhance the production of both butyric acid and lactic acid.

As another embodiment, a method for enhancing the production of butyric acid or lactic acid using one or more selected from the group consisting of dark soy sauce, a precipitate of dark soy sauce with ethanol, a soy sauce-like seasoning liquid, and a precipitate of a soy sauce-like seasoning liquid with ethanol is also provided.

When the method for enhancing the production of butyric acid or lactic acid is carried out for enhancing the amount of butyric acid in the body (preferably in the bowel), or carried out in production of butyric acid by butyrate-producing bacteria, for example, the production of butyric acid by butyrate-producing bacteria can be enhanced. In the case where the method for enhancing the production of butyric acid or lactic acid is carried out for enhancing the amount of butyric acid in the body, the method may include a process of administering the butyric acid or lactic acid production enhancer in the present embodiment to a target. The administration target is preferably a mammal, more preferably a human, a monkey, a cat, a pig, a horse, a cattle, a mouse, a rat, a guinea pig, a dog or a rabbit, and further more preferably a human. The dosage amount and the administration frequency of the butyric acid or lactic acid production enhancer may be appropriately set according to a route of administration, and various conditions such as an age, a body weight and a symptom of the administration target.

In the present specification, the method includes a therapeutic method and a non-therapeutic method, and the use includes therapeutic use and non-therapeutic use.

As another embodiment, a food composition containing a growth promoter for butyrate-producing bacteria may be understood as a food composition containing butyric acid or lactic acid production enhancer. That is, the food composition containing one or more selected from the group consisting of dark soy sauce, a precipitate of dark soy sauce with ethanol, a soy sauce-like seasoning liquid, and a precipitate of a soy sauce-like seasoning liquid with ethanol may be a food composition containing a growth promoter for butyrate-producing bacteria, a food composition containing a butyric acid or lactic acid production enhancer, or both.

In the present embodiment, the food composition may be a food composition for enhancing the production of butyric acid or lactic acid, which contains butyric acid or lactic acid. The food composition containing a butyric acid or lactic acid production enhancer may be food composition for enhancing the production of butyric acid or lactic acid, which contains a growth promoter for butyrate-producing bacteria.

The dosage form of the growth promoter for butyrate-producing bacteria, or the butyric acid or lactic acid production enhancer is not particularly limited, but may be in a dosage form of an oral preparation such as a tablet, a capsule, a granule, a subtle granule, a powder, a solution, a syrup, a chewable or a lozenge, an ointment, a gel, a cream, an injection, a sublingual formulation, an inhalant, a suppository or the like. For these dosage forms, heretofore known additives can be used on manufacturing. Although there is no particular limitation, for example, an excipient, a binder, a diluent, a disintegrant, a buffer, a thickener, a stabilizer, an emulsifier, a dispersant, a suspending agent and/or an antiseptic agent which are known in the industry may be used as necessary.

The butyric acid or lactic acid production enhancer, or the growth promoter for butyrate-producing bacteria may be blended in food, feed, medicaments and the like.

The content of one or more selected from the group consisting of dark soy sauce, a precipitate of dark soy sauce with ethanol, a soy sauce-like seasoning liquid and a precipitate of a soy sauce-like seasoning liquid with ethanol in the growth promoter for butyrate-producing bacteria or the butyric acid or lactic acid production enhancer is not limited as long as a relevant function can be exhibited. The content is preferably 0.1 mass% or more, and may be, for example, 0.1 mass%, 0.2 mass%, 0.5 mass%, 1.0 mass%, 2.0 mass%, 3.0 mass%, 4.0 mass%, 5.0 mass%, or 10 mass% or more, based on the total amount of the growth promoter for butyrate-producing bacteria or the butyric acid or lactic acid production enhancer.

The food composition can be made into, for example, a form suitable for a mode of use, and may be in a dosage form of an oral preparation such as a tablet, a capsule, a granule, a subtle granule, a powder, a solution, a syrup, a chewable or a lozenge, an ointment, a gel, a cream, an injection, a sublingual formulation, an inhalant, a suppository or the like although there is no particular limitation. Although there is no particular limitation, for example, the food composition can be made into an edible form such as a solid form, a liquid form, a granulated form, a granular form, a powder form, a capsule form, a cream form, a paste form or a jelly form. When the food composition is made into a capsule form, it may be covered with one or more layers of film as necessary. For making the food composition into such a dosage form or form, heretofore known additives can be used for manufacturing. Although there is no particular limitation, for example, an excipient, a binder, a diluent, a disintegrant, a buffer, a thickener, a stabilizer, an emulsifier, a dispersant, a suspending agent and/or an antiseptic agent which are known in the food industry may be used as necessary.

The food composition is not particularly limited, but examples thereof include food and drink, health food, functional food, food for specified health use, food with nutrient function claims, health supplements, and supplements.

Examples of the food and drink include beverages, food, seasoning agents, and food additives, specific examples of the beverage include soft drink, fruit juice drink, carbonated drink, milk beverages, alcohol drink, sports drink, and nutritional drink, and specific examples of the food include bread, noodles, rice, soup, prepared food, bean curd, dairy products, and confectionery. Specific examples of the seasoning agent include those used for applications such as bean paste, soy sauce or dressing.

To the food composition, for example, a coloring agent, a flavoring agent, a sweetener, a bittering agent, a sour agent, a preservative, an antioxidant, a pH adjuster, and/or a thickening agent may be added as necessary.

The food composition may be provided after being sterilized by heating, and may be provided in the form of being sealed in a bag or a container. The food composition may be provided in the form of being labeled as "enhance barrier function of bowel", "enhance butyrate-producing bacteria in bowel", "grow butyrate-producing bacteria in bowel" or "suited to people who are concerned about bowel condition".

The food composition may preferably contain an arbitrary amount of a growth promoter for butyrate-producing bacteria, or a butyric acid or lactic acid production enhancer as long as a relevant function can be exhibited.

### Examples

Hereinafter, the present invention will be described in more detail by showing specific examples. Note that the present invention is not limited to these examples.

### <Example 1> Evaluation of effect of dark soy sauce or precipitate of dark soy sauce with ethanol on proliferation of butyrate-producing bacteria

### (1) Preparation of modified YCFA medium

YCFA medium for use in culture of butyrate-producing bacteria and preparation of the following dark soy sauce or precipitate of dark soy sauce with ethanol was prepared by following Tables 1-1 to 1-6 below. The prepared YCFA medium was adjusted to a pH of 7.45 with 10 N NaOH, and then sterilized in an autoclave at 121°C for 20 minutes. Thereafter, the medium sterilized in the autoclave was brought back to room temperature, and 0.5 mL of Vitamin Solution II (Table 1-7) was added.

Subsequently, a preculture solution obtained by culturing butyrate-producing bacteria Agathobacter rectalis JCM 17463^{T} strain (hereinafter, referred to as A. rectalis) purchased from Riken BioResource Research Center was inoculated in the YCFA medium at 2% (v/v), and subjected to stationary culture at 37°C for 16 hours under a constant hydrogen concentration condition in an oxygen-free anaerobic chamber (manufactured by Coy Laboratory Products, Inc.). The resulting culture solution was centrifuged at 7,500 rpm (5,100 × g) for 10 minutes, and the supernatant was sterilized by filtration twice with a 0.20 µm Minisart (registered trademark) syringe filter (manufactured by Sartorius AG) to obtain modified YCFA medium.

The YCFA medium and modified YCFA medium prepared as described above were also used in Example 2 and thereafter.

**Table 1-1 YCFA medium 500 mL**

| [Table 1-1] | |
|---|---|
| Component name | Weight or volume |
| Tryptone | 5.00 g |
| Yeast extract | 1.25 g |
| NaHCO₃ | 2.00 g |
| Glucose | 1.00 g |
| Maltose | 1.00 g |
| Cellobiose | 1.00 g |
| L-cysteine.Hcl.H₂O | 0.50 g |
| Resazurin | 0.5 mg |
| Mineral solution I | 75.0 mL |
| Mineral solution II | 75.0 mL |
| VFA mix | 3.1 mL |
| Hemin solution | 5.0 mL |
| Vitamin solution I | 0.5 mL |
| Distilled water | 330.0 mL |

**Table 1-2 Mineral solution I**

| [Table 1-2] | |
|---|---|
| Component name | Weight or volume |
| K₂HPO₄ | 0.225 g |
| Distilled water | 75.00 mL |

**Table 1-3 Mineral solution II**

| [Table 1-3] | |
|---|---|
| Component name | Weight or volume |
| KH₂PO₄ | 0.225 g |
| (NH₄) ₂SO₄ | 0.450 g |
| NaCl | 0.450 g |
| MgSO₄·7H₂O | 0.045 g |
| CaCl₂·2H₂O | 0.045 g |
| Distilled water | 75.00 mL |

**Table 1-4 VFA (volatile fatty acid) mix**

| [Table 1-4] | |
|---|---|
| Component name | Volume |
| Acetic acid | 17.0 mL |
| Propionic acid | 6.0 mL |
| n-Valeric acid | 1.0 mL |
| iso-Valeric acid | 1.0 mL |
| iso-Butyric acid | 1.0 mL |
| 10N NaOH | 26.0 mL |

**Table 1-5 Hemin solution**

| [Table 1-5] | |
|---|---|
| Component name | Weight or volume |
| potassium hydroxide | 0.14 g |
| Hemin | 0.05 g |
| Ethanol | 12.5 mL |
| Distilled water | 37.5 mL |

**Table 1-6 Vitamin Solution I**

| [Table 1-6] | |
|---|---|
| Component name | Weight or volume |
| Biotin | 0.5 mg |
| Vitamin B12 | 0.5 mg |
| p-Aminobenzoic acid | 1.5 mg |
| Folic acid | 2.5 mg |
| Pyridoxine·HCl | 7.5 mg |
| Distilled water | 50.0 mL |

**Table 1-7 Vitamin solution II**

| [Table 1-7] | |
|---|---|
| Component name | Weight or volume |
| Thiamine·HCl | 2.5 mg |
| Riboflavin | 2.5 mg |
| Distilled water | 50.0 mL |

### (2) Preparation of precipitate of dark soy sauce with ethanol

To dark soy sauce (manufactured by KIKKOMAN CORPORATION) was added three times its amount of 99.5% ethanol, and a precipitate generated was dissolved in Milli-Q water. The solution containing the precipitate was dialyzed against Milli-Q water for 24 hours using 10 mL of Spectra/Por (registered trademark) Float-A-Lyzer (registered trademark) G2CE, 100D-500D (manufactured by Repligen Corporation). The solution after dialysis was freeze-dried for 48 hours with a bench shelf-type vacuum freeze drier AdVantage Pro series (manufactured by SP Scientific) to obtain a precipitate of dark soy sauce with ethanol. The dried powder was weighed, and dissolved in the modified YCFA medium, and the solution was sterilized by filtration with a 0.22 µm PES syringe filter (manufactured by OSAKA CHEMICAL Co., Ltd.), and used in (3) below.

### (3) Evaluation of effect of dark soy sauce or precipitate of dark soy sauce with ethanol on proliferation of A. rectalis

A. rectalis stored at -80°C as a glycerol stock was thawed in an anaerobic chamber (manufactured by Coy Laboratory Products, Inc.), diluted 5-fold with the YCFA medium, and subjected to stationary culture overnight at 37°C under a constant hydrogen concentration condition to perform restoration culture. The resulting restoration culture solution was diluted 10-fold with the YCFA medium, and subjected to successive culture, and the resulting culture solution was diluted with modified YCFA medium so that OD₆₀₀ was within a range of 0.15 to 0.25, thereby preparing a successive culture solution.

The successive culture solution was inoculated in an amount of 50 µL in 950 µL of modified YCFA medium containing dark soy sauce or a precipitate of dark soy sauce with ethanol at a predetermined concentration, or modified YCFA medium free of dark soy sauce or a precipitate of dark soy sauce with ethanol as a control, and subjected to stationary culture at 37°C for 16 hours under a constant hydrogen concentration condition. After the stationary culture, OD₆₀₀ of the culture solution was measured.

Using, as an index, a value (proliferative activity) obtained by dividing the value of OD₆₀₀ of the culture solution subjected to stationary culture as described above using modified YCFA medium containing dark soy sauce or a precipitate of dark soy sauce with ethanol at a predetermined concentration, by the value of OD₆₀₀ of the culture solution (control) subjected to stationary culture as described above using modified YCFA medium free of dark soy sauce or a precipitate of dark soy sauce with ethanol, the effect of dark soy sauce or a precipitate of dark soy sauce with ethanol on proliferation of A. rectalis was evaluated. The results of the above evaluation are shown in Tables 2-1 and 2-2.

**Table 2-1 Results of proliferative activity in 25.0 mg/mL precipitate of dark soy sauce with ethanol**

| [Table 2-1] | |
|---|---|
| Concentration of precipitate with ethanol (mg/mL) | Proliferative activity |
| 0 (Control) | 1.00 |
| 25.0 | 1.34 |

**Table 2-2 Results of proliferative activity in 5.0% (v/v) dark soy sauce**

| [Table 2-2] | |
|---|---|
| Concentration of dark soy sauce (% (v/v)) | Proliferative activity |
| 0 (Control) | 1.00 |
| 5.0 | 1.23 |

### <Example 2> Evaluation of effect of soy sauce-like seasoning liquid or precipitate of soy sauce-like seasoning liquid with ethanol on proliferation of butyrate-producing bacteria and production of n-butyric acid and lactic acid

### (1) Preparation of precipitate of soy sauce-like seasoning liquid with ethanol

To a soy sauce-like seasoning liquid (manufactured by KIKKOMAN CORPORATION) was added three times its amount of 99.5% ethanol, and a precipitate generated was dissolved in Milli-Q water. The solution containing the precipitate was dialyzed against Milli-Q water for 24 hours using 10 mL of Spectra/Por (registered trademark) Float-A-Lyzer (registered trademark) G2CE, 100D-500D (manufactured by Repligen Corporation). The solution after dialysis was freeze-dried for 48 hours with a bench shelf-type vacuum freeze drier AdVantage Pro series (manufactured by SP Scientific) to obtain a precipitate of a soy sauce-like seasoning liquid with ethanol. The dried powder was weighed, and dissolved in the modified YCFA medium, and the solution was sterilized by filtration with a 0.22 µm PES syringe filter (manufactured by OSAKA CHEMICAL Co., Ltd.), and used in (2) below.

### (2) Evaluation of effect of soy sauce-like seasoning liquid or precipitate of soy sauce-like seasoning liquid with ethanol on proliferation of A. rectalis and production of n-butyric acid and lactic acid

A. rectalis stored at -80°C as a glycerol stock was thawed in an anaerobic chamber (manufactured by Coy Laboratory Products, Inc.), diluted 5-fold with the YCFA medium, and subjected to stationary culture overnight at 37°C under a constant hydrogen concentration condition to perform restoration culture. The resulting restoration culture solution was diluted 10-fold with the YCFA medium, and subjected to successive culture, and the resulting culture solution was diluted with modified YCFA medium so that OD₆₀₀ was within a range of 0.15 to 0.25, thereby preparing a successive culture solution.

The successive culture solution was inoculated in an amount of 50 µL in 950 µL of modified YCFA medium containing a soy sauce-like seasoning liquid or a precipitate of a soy sauce-like seasoning liquid with ethanol at a predetermined concentration, or modified YCFA medium free of a soy sauce-like seasoning liquid or a precipitate of a soy sauce-like seasoning liquid with ethanol as a control, and subjected to stationary culture at 37°C for 15 hours under a constant hydrogen concentration condition. After the stationary culture, OD₆₀₀ of the culture solution was measured.

The supernatant of the culture solution subjected to stationary culture was centrifuged at 15,000 rpm (204 × 100 g) and 4°C for 10 minutes. The centrifugal supernatant was sterilized by filtration with 0.22 µm Millex (registered trademark) - GV, PVDF (manufactured by Merck KGaA), and added to 500 µL in volume of Amicon (registered trademark) Ultra Ultracel-10K-0.5 mL (manufactured by Merck KGaA), and the mixture was centrifuged at 12,900 rpm (150 × 100 g) and 4°C for 10 minutes to perform ultrafiltration. The flow-through solution was collected, and the amounts of n-butyric acid and lactic acid were measured by a pH buffering-electric conductivity detection method (Shimadzu high-performance liquid chromatograph, organic acid analysis system, applied data collection) using a Nexera organic acid analysis system (Shimadzu Corporation). For separating n-butyric acid and lactic acid, three Shim-pack SCR-102H (300 mm × 8 mm I.D. 7 µm) culumns were connected in series (temperature 50°C), and a 2.5 mmol/L p-toluenesulfonic acid aqueous solution (flow rate 0.8 mL/min) was used as an eluting solution. For detection, an eluate from the column was mixed with 2.5 mmol/L p-toluenesulfonic acid, 10 mmol/L Bis-Tris and a 0.05 mmol/L EDTA-4H buffer solution (flow rate 0.8 mL/min), and an electrical conductivity meter (polatity: +) was used.

Using, as an index, a value (proliferative activity) obtained by dividing the value of OD₆₀₀ of the culture solution subjected to stationary culture as described above using modified YCFA medium containing a soy sauce-like seasoning liquid or a precipitate of a soy sauce-like seasoning liquid with ethanol at a predetermined concentration, by the value of OD₆₀₀ of the culture solution (control) subjected to stationary culture as described above using modified YCFA medium free of a soy sauce-like seasoning liquid or a precipitate of a soy sauce-like seasoning liquid with ethanol, the effect of a soy sauce-like seasoning liquid or a precipitate of a soy sauce-like seasoning liquid with ethanol on proliferation of A. rectalis was evaluated. The effect of a soy sauce-like seasoning liquid or a precipitate of a soy sauce-like seasoning liquid with ethanol on the production of n-butyric acid and lactic acid was similarly evaluated using, as an index, a value (production amount ratio for each of n-butyric acid and lactic acid) obtained by dividing the value of the amount of each of n-butyric acid and lactic acid measured by the HPLC method, by the value for the control. The results of the above evaluation are shown in Tables 2-1 and 2-2.

Table 3-1 Results of proliferative activity and production amount ratios for n-butyric acid and lactic acid at each concentration of precipitate of soy sauce-like seasoning liquid with ethanol

**[Table 3-1]**

| Concentration of precipitate with ethanol (mg/mL) | Proliferative activity | Production amount ratio | |
|---|---|---|---|
| | | n-Butyric acid | Lactic acid |
| 0 (Control) | 1.00 | 1.00 | 1.00 |
| 2.5 | 1.26 | 1.29 | 1.20 |
| 5.0 | 1.57 | 1.68 | 1.42 |
| 10.0 | 2.10 | 2.50 | 2.02 |
| 25.0 | 3.50 | 5.00 | 4.05 |

Table 3-2 Results of proliferative activity and production amount ratio for n-butyric acid and lactic acid in 5.0% (v/v) soy sauce-like seasoning liquid

**[Table 3-2]**

| Concentration of soy sauce-like seasoning liquid (% (v/v)) | Proliferative activity | Production amount ratio | |
|---|---|---|---|
| | | n-Butyric acid | Lactic acid |
| 0 (Control) | 1.00 | 1.00 | 1.00 |
| 5.0 | 2.63 | 3.30 | 1.50 |

### <Comparative Example 1> Evaluation of effect of inulin on proliferation of A. rectalis and production of n-butyric acid and lactic acid

Table 3 shows the results of evaluating proliferative activity for A. rectalis and production amount ratios for n-butyric acid and lactic acid in the same manner as in Example 2 using modified YCFA medium containing inulin (manufactured by Fuji Nihon Seito Corporation) at a predetermined concentration. The values of the proliferative activity and the production amount ratios for n-butyric acid and lactic acid were larger in Example 2 with a soy sauce-like seasoning liquid or a precipitate of a soy sauce-like seasoning liquid with ethanol, and the soy sauce-like seasoning liquid or the precipitate of a soy sauce-like seasoning liquid with ethanol were found to have a larger effect on proliferation of butyrate-producing bacteria and the production of n-butyric acid and lactic acid.

Table 4 Results of proliferative activity and production amount ratios for n-butyric acid and lactic acid at each concentration of inulin

**[Table 4]**

| Concentration of inulin (mg/mL) | Proliferative activity | Production amount ratio | |
|---|---|---|---|
| | | n-Butyric acid | Lactic acid |
| 0 (Control) | 1.00 | 1.00 | 1.00 |
| 2.5 | 1.09 | 0.98 | 0.99 |
| 5.0 | 1.16 | 0.86 | 0.89 |
| 10.0 | 1.21 | 1.20 | 1.27 |
| 25.0 | 1.38 | 1.41 | 1.54 |

### <Example 3> Evaluation of components of precipitate of soy sauce-like seasoning liquid with ethanol

The infrared absorption spectrum of powder obtained by freeze-drying a precipitate of a soy sauce-like seasoning liquid with ethanol was measured by Fourier transform infrared spectroscopy (FTIR) using a Fourier transform infrared spectrophotometer (IRAffinity-1S, Shimadzu Corporation) equipped with a deuterated L-alanine-doped triglycine sulfate (DLaTGS) detector. The measurement was performed using a transmission method, the measurement wavenumber range was from 400 to 4000 cm⁻¹, the resolution was 4 cm⁻¹, and scanning was performed 45 times.

As a result, peaks at 3279 cm⁻¹, 1584 cm⁻¹, 1402 cm⁻¹ and 1042 cm⁻¹, which are derived from a hydroxyl group, a carbonyl group, C-H and an ether group, respectively, were detected, and it was suggested that the main components of the precipitate of a soy sauce-like seasoning liquid with ethanol were saccharides (Figure 1).

Further, for the precipitate of a soy sauce-like seasoning liquid with ethanol, analysis of monosaccharides was performed by referring to a published liquid chromatography method (HPLC Chromaster - Introduction to Analysis of Saccharides (Chromaster Phosphoric Acid-Phenylhydrazine Post Column Method), Hitachi High-Tech Corporation. https://www.hitachi-hightech.com/jp/ja/knowledge/analytical-systems/hplc/chromaster-ultra/cm-data26.html). As a result, monosaccharides or disaccharides were not detected. From the results of Example 3 above, it was confirmed that the precipitate of a soy sauce-like seasoning liquid with ethanol according to the invention of the present application mainly contained polysaccharides.

## Claims

1. A growth promoter for butyrate-producing bacteria, comprising dark soy sauce or a soy sauce-like seasoning liquid, or a precipitate thereof with ethanol.

2. The growth promoter for butyrate-producing bacteria according to claim 1, comprising a polysaccharide derived from dark soy sauce or a soy sauce-like seasoning liquid.

3. A food composition comprising the growth promoter for butyrate-producing bacteria according to claim 1 or 2.

4. A food composition for promoting growth of butyrate-producing bacteria, comprising dark soy sauce or a soy sauce-like seasoning liquid, or a precipitate thereof with ethanol.

5. A method for promoting growth of butyrate-producing bacteria using dark soy sauce or a soy sauce-like seasoning liquid, or a precipitate thereof with ethanol.

6. A butyric acid or lactic acid production enhancer comprising dark soy sauce or a soy sauce-like seasoning liquid, or a precipitate thereof with ethanol.

7. The butyric acid or lactic acid production enhancer according to claim 6, comprising a polysaccharide derived from dark soy sauce or a soy sauce-like seasoning liquid.

8. A food composition comprising the butyric acid or lactic acid production enhancer according to claim 6 or 7.

9. A food composition for enhancing the production of butyric acid or lactic acid, comprising dark soy sauce or a soy sauce-like seasoning liquid, or a precipitate thereof with ethanol.

10. A method for enhancing the production of butyric acid or lactic acid using dark soy sauce or a soy sauce-like seasoning liquid, or a precipitate thereof with ethanol.
